(19) Europäisches Patentamt
European Patent Office
Office européen des brevets

(11) Publication number : **0 445 987 A2**

# (12) EUROPEAN PATENT APPLICATION

(21) Application number : **91301721.6**

(51) Int. Cl.⁵ : **C07D 211/90**, A61K 31/445

(22) Date of filing : **01.03.91**

(30) Priority : **07.03.90 JP 55860/90**
**02.04.90 JP 87713/90**
**14.02.91 JP 40770/91**

(43) Date of publication of application :
**11.09.91 Bulletin 91/37**

(84) Designated Contracting States :
**BE CH DE FR GB LI**

(71) Applicant : **Higashikawa, Tetsuro**
**21-27-902, Shimomeguro-2-chome**
**Meguro-ku, Tokyo (JP)**

(72) Inventor : **Yonemura, Tomohiko**
**892, Kagamimachi**
**Yatsushiro-gun, Kumamoto-ken (JP)**

(74) Representative : **Perry, Robert Edward et al**
**GILL JENNINGS & EVERY 53-64 Chancery Lane**
**London WC2A 1HN (GB)**

## (54) Novel process for producing 2,6-dimethyl-4-(m-nitrophenyl)-1,4-dihydropyridine-3,5-dicarboxylate-3-methyl ester-5-beta-(N-benzyl-N-methylamino)ethyl ester.

(57) Nicardipine hydrochloride, i.e. 2,6-dimethyl-4-(m-nitrophenyl)-1,4-dihydropyridine-3,5-dicarboxylate 3-methyl ester-5-β-(N-benzyl-N-methylamino)ethyl ester, is prepared in a short time at a high yield by reacting, in a non-aqueous system, m-nitrobenzaldehyde or another compound of formula (1)

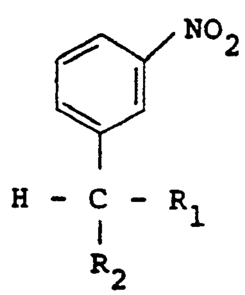

(1)

wherein either $R_1$ and $R_2$ are taken together and represent =O or =N-lower alkyl, or $R_1$ represents -O-, $R_2$ represents -NH-, and $R_1$ and $R_2$ are linked via $C_{2-4}$ straight or branched-chain alkylene to form a saturated heterocyclic ring which may be substituted, simultaneously or sequentially, in either order, with methyl 3-aminocrotonate and (N-methyl-N-benzylamino)ethyl 3-aminocrotonate.

EP 0 445 987 A2

# NOVEL PROCESS FOR PRODUCING 2,6-DIMETHYL-4-(m-NITROPHENYL)-1,4-DIHYDROPYRIDINE-3,5-DICARBOXYLATE-3-METHYL ESTER-5-β-(N-BENZYL-N-METHYLAMINO)ETHYL ESTER

## BACKGROUND OF THE INVENTION

### FIELD OF THE INVENTION

The present invention relates to a process for producing 2,6-dimethyl-4-(m-nitrophenyl)-1,4-dihydropyridine-3,5-dicarboxylate-3-methyl ester-5-β-(N-benzyl-N-methylamino)ethyl ester.hydrochloride represented by the formula (2):

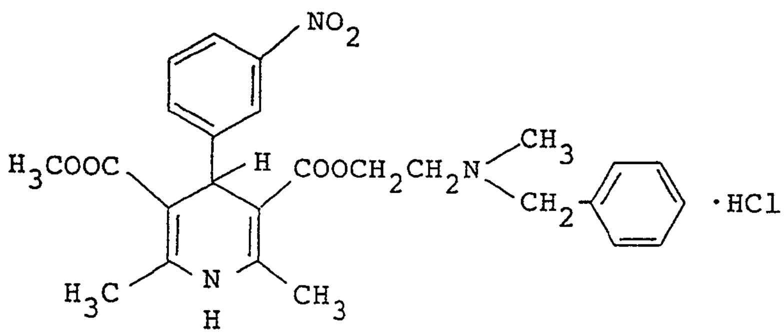

... (2)

This compound (hereinafter abbreviated to nicardipine hydrochloride) is useful as a hypotensive agent and a vasodilator.

### DESCRIPTION OF THE PRIOR ART

Nicardipine hydrochloride and processes for synthesizing the compound are known. The conventional process predominantly used for synthesis of nicardipine hydrochloride is a process comprising reacting an acetoacetic acid ester with m-nitrobenzaldehyde in an aqueous medium such as isopropyl alcohol or the like to prepare a 2-(m-nitrobenzylidene)acetoacetic acid ester and condensing the ester with a 3-aminocrotonic acid ester, or reacting simultaneously an acetoacetic acid ester, m-nitrobenzaldehyde and a 3-aminocrotonic acid ester, i.e. a process using an acetoacetic acid ester as one of starting materials (Japanese Patent Publication Nos. 45075/1980 and 6417/1981). Other processes have problems in practical application because they, for example, need more steps than the above process.

As appreciated from the above formula (2), in nicardipine, two carboxylic groups have different alkoxy groups; therefore, consideration is needed in its synthesis. Besides, the conventional processes require a long reaction time and give a low yield.

The present inventors made study on an improved process for synthesis of nicardipine and found that surprisingly nicardipine can be synthesized at a high yield in a short time without using acetoacetic acid ester and reacting a compound represented by the following general formula (1)

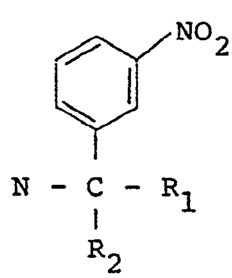

(1)

wherein $R_1$ and $R_2$ taken together represent =O and =N-lower alkyl or when $R_1$ represents -O- and $R_2$ represents -NH-, $R_1$ and $R_2$ together form a saturated hetero ring which may be substituted, via $C_2$-$C_4$ straighted or branched alkylene, with methyl 3-aminocrotonate and (N-methyl-N-benzylamino)ethyl 3-aminocrotonate simultaneously or in an optional order.

Therefore, the object of the present invention is to provide a novel process for synthesize nicardipine by reacting a compound represented by the general formula (1) with two 3-aminocrotonic acid esters mentioned above.

## SUMMARY OF THE INVENTION

The present invention resides in a process for producing nicardipine easily by reacting a compound represented by the general formula (1) with methyl 3-aminocrotonate and (N-methyl-N-benzylamino)ethyl 3-aminocrotonate simultaneously or in an optional order.

## DESCRIPTION OF THE PREFERRED EMBODIMENTS

The present invention is hereinafter described in detail.

The compound of the general formula (1) used in the process of the present invention include specifically, for example, m-nitrobenzaldehyde, 2-(m-nitrophenyl)-4,4-dimethyloxazolidine, oxazolidine of 2-(m-nitrophenyl)-tetrahydro-(2H)-1,3-oxazine, tetrahydro-(2H)-1,3-oxazines and N-alkyl-m-nitrobenzaldimine.

In the present invention, a desirably equimolar mixture of the above reaction components is subjected to a reaction in a non-aqueous solvent in the presence of an acid, whereby an intended condensate can be obtained easily.

As the solvent used in the process of the present invention, there is preferred a basic solvent (e.g. pyridine, picoline, aniline, dimethylaniline), dimethylformamide, acetonitrile, etc., or their mixture, to an alcoholic solvent. In order to allow the reaction to proceed more smoothly, the presence of an acid in a catalytic or excessive amount is effective. The acid includes small amounts of hydrogen chloride (this is added as, for example, pyridine hydrochloride) and organic acids (e.g. benzenesulfonic acid, acetic acid, trifluoroacetic acid). In the present invention, the yield is generally good though it is affected in some degree by the rate or order of aminocrotonic acid esters added.

### Example 1

1.51 g of m-nitrobenzaldehyde was dissolved in 20 ml of anhydrous pyridine and 0.1 ml of acetic acid was added therein. The resulting mixture was heated to 70°C and 2.48 g of (N-methyl-N-benzylamino)ethyl aminocrotonate and 1.15 g of methyl 3-aminocrotonate were gradually added in it. The resulting mixture was heated for about 1 hour. After the completion of the reaction, pyridine was removed by distillation under reduced pressure. The resulting product was dissolved in methylene chloride. The solution was extracted with 1 N hydrochloric acid to obtain a basic portion. It was neutralized with $NaHCO_3$ and then reextracted with methylene chloride. The extract was water-washed, dehydrated, and then methylene chloride was distilled off. The resulting product was purified by silica gel chromatography. The purified product was dissolved in hydrochloric acid/methanol, and the solution was concentrated to dryness under reduced pressure. The residue was recrystallized as a hydrochloride from methanol/acetone to obtain an intended compound having a melting point of 166-167°C at a yield of 47%. The compound was identified to be the same as the standard nicardipine, by NMR.

### Example 2

To 225 ml of pyridine and 33.75 ml of acetic acid in a 500-ml flask were added 11.325 g of m-nitrobenzaldehyde, 12.4 g of (N-methyl-N-benzylamino)ethyl 3-aminocrotonate and 8.625 g of methyl aminocrotonate (a product of Aldrich Chemical Co., Inc., lot No. 04505HT). The mixture was heated under refluxing at 125°C for 2 hours. After the completion of the reaction, the solvent was removed by distillation under reduced pressure. The residue was mixed with 200 ml of toluene and the solvent was removed by distillation under reduced pressure. The residue was mixed with 500 ml of chloroform and the resulting solution was washed with 300 ml of diluted hydrochloric acid of pH 4.0. The solvent was removed by distillation under reduced pressure. The residue was subjected to column chromatography (column = 5 cm in diameter and 60 cm in height, silica gel = 350 g of BW-820 MH manufactured by Fuji Davison Chemical Co.) and 12.8 g of a solid was obtained from a fraction eluting with a chloroform/acetone (20:1) while the formation of the desired compound being identified with thin-layer column chromatography (Kieselguhr 60F254 manufactured by Merck Co., 1.5 x 6.6 cm, detection by UV). The solid was dissolved in 300 ml of chloroform and the solution was washed with 1,000 ml of a citric acid/disodium hydrogenphosphate buffer solution of pH 4. The solvent was then removed by distillation under reduced pressure to obtain 11.2 g of a solid which was dissolved in 200 ml of methanol. The resulting solution

was cooled to 10°C and hydrogen chloride gas was incorporated thereinto. Methanol was removed by distillation under reduced pressure. To the residue was added 40 ml of acetone, and the mixture was stirred with ice cooling, to obtain 5.8 g of yellow crystals as primary crystals.

The crystals have a melting point of 166-169°C. Their analysis results on IR spectrum and NMR spectrum agreed with those of the standard nicardipine hydrochloride.

Testers used for analysis

1. Melting point tester Yanaco Model MP manufactured by Yanagimoto Seisakusho K-K.
2. IR spectrum tester Jasco Model A-100 manufactured by Nihon Bunko Kogyo K-K.
3. NMR spectrum tester Hitachi R-120 manufactured by Hitachi, Ltd.

Example 3

192 g of m-nitrobenzaldehyde, 300 g of (N-methyl-N-benzylamino)ethyl 3-aminocrotonate and 146 g of methyl 3-aminocrotonate were added to a mixture consisting of 3,810 ml of pyridine was 572 ml of acetic acid. The mixture was refluxed at 118-124°C for 2 hours. The solvent was removed by distillation under reduced pressure. The residue was dissolved in 2,170 ml of chloroform and the solution was washed with 1 N hydrochloric acid, water and 3% sodium carbonate. Each of the three washings was extracted with 1,000 ml of chloroform. Each extract was combined with the solution and washed again. The resulting chloroform solution was dried over sodium sulfate, and subjected to vacuum distillation to remove the solvent. The resulting residue was dissolved in acetone and purified, and recrystallized from acetone according to an ordinary method to obtain 160 g of yellow crystals of nicardipine hydrochloride as primary crystals. Melting point = 169°C

Example 4

192 g of m-nitrobenzaldehyde, 300 g of (N-methyl-N-benzylamino)ethyl 3-aminocrotonate and 146 g of methyl 3-aminocrotonate were added to 3,810 g of anhydrous pyridine containing 572 mg of acetic anhydride. The mixture was subjected to reaction at 118-124°C for 6 hours with stirring.

After the completion of the reaction, the same operation as in Example 1 was effected to obtain 140 g of nicardipine hydrochloride having a melting point of 169°C.

Example 5

In 50 ml of triacetonitrile were dissolved 2.4 g of 2-(m-nitrophenyl)-4,4-dimethyloxazolidine, 2.48 g of (N-methyl-N-benzylamino)ethyl 3-aminocrotonate and 1.15 g of methyl 3-aminocrotonate. 2 ml of acetic acid was added thereto, and the mixture was refluxed with heating. After the disappearance of the raw materials was confirmed by thin-layer chromatography, heating was effected for 1 hour. The solvent was removed by distillation and then, the same operation as in Example 1 was effected to obtain an intended compound at an yield of about 35%. The reaction time was about 2 hours in total.

Example 6

To 225 ml of pyridine and 33.75 ml of acetic acid in a 500-ml flask were added 16.65 g of 2-(m-nitrophenyl)-4,4-dimethyl-oxazolidine, 12.4 g of (N-methyl-N-benzylamino)ethyl 3-aminocrotonate was 8.625 g of methyl 3-aminocrotonate (a product of Aldrich Chemical Co., Inc., lot No. 04505HT). The mixture was heated for 2 hours under refluxing at 125°C. After the completion of the reaction, the solvent was removed by distillation under reduced pressure, 200 ml of toluene was added to the residue and the solvent was removed by distillation under reduced pressure, 500 ml of chloroform was added to the residue, and the resulting solution was washed with 300 ml of diluted hydrochloric acid of pH 4.0. The solvent was further removed by distillation under reduced pressure. The residue was subjected to column chromatography (column = 5 cm in diameter and 60 cm in height, silica gel = 350 g of BW-820MH manufactured by Fuji Davison Chemical Co.) and 10.5 g of a solid was obtained from a fraction eluting with chloroform/acetone (20:1) while the formation of the desired compound being identified with thin-layer column chromatography (Kieselguhr 60F254 manufactured by Merck Co., 1.5 x 6.6 cm, detection by UV). The solid was dissolved in 300 ml of chloroform and the solution was washed with 1,000 ml of a citric acid/disodium hydrogenphosphate buffer solution of pH 4. The solvent was removed by distillation under reduced pressure to obtain 8.8 g of a solid which was dissolved in 200 ml of methanol. The resulting solution was cooled to 10°C and hydrogen chloride gas was incorporated thereinto. Methanol was removed

by distillation under reduced pressure. To the residue was added 40 ml of acetone, and the mixture was stirred with the cooling, to obtain 5.3 g of yellow crystals as primary crystals.

The crystals had a melting point of 167-169°C. Their analysis results on IR spectrum and NMR spectrum agreed with those of the standard nicardipine hydrochloride.

Example 7

The same procedure as in Example 1 was repeated except that the m-nitrobenzaldehyde (192 g) was replaced by 2-(m-nitrophenyl)-4,4-dimethyloxazolidine (267 g) and that picoline was used as the solvent, whereby 150 g of nicardipine hydrochloride were obtained.

Example 8

In 100 ml of acetonitrile was dissolved 1.68 g of N-methyl-m-nitrobenzaldemine, 2.48 g of (N-methyl-N-benzylamino)ethyl 3-aminocrotonate was 1.15 g of methyl 3-aminocrotonate. 1 ml of acetic acid was added thereto, and the mixture was refluxed for 1 hour. Refluxing was continued for a further 10 hours for safety and then acetonitrile was removed. The residue was subjected to the same treatment as in Example 1 to obtain an intended compound at a yield of 40%.

Example 9

The same procedure as in Example 6 was repeated except that 2-(m-nitrophenyl)-4,4-dimethyloxazolidine (16.65 g) was replaced by 2-(m-nitrophenyl)-tetrahydro-(2H)-1,3-oxazine (15-53 g ), to obtain 5-5 g of nicardipine hydrochloride as primary crystals.

According to the present invention, Nicardipine was be obtained at a high yield in a relatively short time.

## Claims

1. A process for producing 2,6-dimethyl-4-(m-nitrophenyl)-1,4-dihydropyridine-3,5-dicarboxylate 3-methyl ester 5-β-(N-benzyl-N-methylamino)ethyl ester, wherein, in a non-aqueous system, a compound of formula (1)

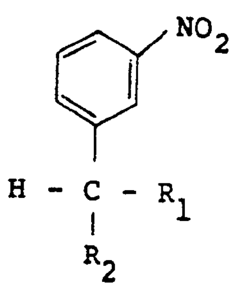

(1)

wherein either $R_1$ and $R_2$ are taken together and represent =O or =N-lower alkyl, or $R_1$ represents -O-, $R_2$ represents -NH-, and $R_1$ and $R_2$ are linked via $C_{2-4}$ straight or branchedchain alkylene to form a saturated heterocyclic ring which may be substituted, is reacted simultaneously or sequentially, in either order, with methyl 3-aminocrotonate and (N-methyl-N-benzylamino)ethyl 3-aminocrotonate.

2. A process according to claim 1, which is conducted in an anhydrous basic solvent in the presence of an at least catalytic amount of an acid.

3. A process according to claim 2, wherein the basic solvent is pyridine and the acid is acetic acid.